# EUROPEAN PATENT APPLICATION

(11) **EP 3 871 567 A1**
(43) Date of publication of application: **01.09.2021**
(21) Application number: 21397502.2
(22) Date of filing: 24.02.2021
(51) Int. Cl.: A47G 29/14, A47G 29/20, A47G 29/28, E05G 7/00

(54) **PASS-THROUGH CABINET**

(30) Priority: 25.02.2020 FI 20200030 U
(71) Applicant: Tekoniemi, Simo, 04440 Järvenpää (FI)
(72) Inventor: Tekoniemi, Simo, 04440 Järvenpää (FI)
(74) Representative: Salomäki, Juha Kari Ensio

(57) **Abstract**

The invention relates to a pass-through cabinet (1) comprising at least a loading side door (2), an unloading side door (3) and a lock arrangement (5) to lock and unlock the doors (2, 3). The pass-through cabinet (1) is equipped with an indicator system adapted to indicate the operating state of the pass-through cabinet (1).

## Description

The invention relates to a pass-through cabinet as defined in the preamble of claim 1.

### BACKGROUND OF THE INVENTION

The solution according to the invention is applicable in hospitals, health centers and similar facilities, as well as in large-scale events, such as sports competitions in which people give or to which people bring different kinds of samples to be analyzed. A staff member takes a desired sample of a customer, or the customer takes the sample by himself/herself and hand it over to the staff for further analysis.

Privacy is needed for giving certain samples, such as a urine sample, that is, the customer wants to produce the sample in a sample container by himself/herself. For analysis, the sample is handed over to the staff, or, it is put in an indicated place, such as in a cabinet, to be fetched by the staff.

As is known, these points of handover do not have any indicator, such as an indicator light, to indicate to the staff when the sample has been put into the cabinet. As an example, if there are several sample collection cabinets side by side, the customer cannot see which sample loading door or sample collection cabinet is vacant. This is a problem especially in massive events or vast sample collection areas. Neither do the known points of handover have a locked space to receive the sample, allowing outsiders to manipulate the sample or to read personal information, such a social security number, from the side of the sample container.

### OBJECTIVE OF THE INVENTION

The invention aims at eliminating the above-mentioned drawbacks of the prior art and to provide a novel solution making it easier for the staff to observe the presence of a sample, by means of an unambiguous, reliably operating indicator system, such as a system based on indicator lights, protecting the sample from the influence of outsiders. The pass-through cabinet according to the invention, hereafter briefly also referred to as "the cabinet", is characterized in what is set forth in the characterizing part of claim 1. The other embodiments of the invention are characterized in what is set forth in the rest of the claims.

### BRIEF DESCRIPTION OF THE INVENTION

To achieve its objective, the pass-through cabinet according to the invention comprises at least a loading side door, an unloading side door and a lock arrangement for locking and unlocking the doors. Preferably, the pass-through cabinet is equipped with an indicator system adapted to indicate the operating state of the pass-through cabinet.

### ADVANTAGES OF THE INVENTION

An advantage of the solution according to the invention is that the solution is simple, safe and operates reliably. As the loading side door of the pass-through cabinet is locked automatically, after a sample has been loaded into the cabinet, outsiders cannot access the sample and its information. Besides, at the step of giving the sample, as the unloading side door is locked, there is no connection from the loading side to the unloading side through the cabinet. A major advantage of the solution is the indicator system implemented by means of indicator lights, for example. It allows the person giving the sample to know which pass-through cabinet is vacant, and, once the sample has been loaded into the cabinet and the loading side door has been closed, the indicator system of the cabinet indicates, on the unloading side, that there is a sample in the cabinet. Thus, it is easy to detect on the unloading side that a sample has been given. The indicator system is especially useful when the sample collection place has several pass-through cabinets. It is easy to see which of the cabinets contains a sample. Another advantage is that the cabinet is simple in structure and, therefore, inexpensive to purchase. Still another advantage is that the lock arrangement and the control system of the cabinet can be provided in a single unit, as a single module, such as on a single circuit board, jointly replaceable, for example, if the cabinet suffers malfunctions or if it is desirable to the change its operation in some other way.

### LIST OF FIGURES

In the following, the invention will be described in more detail, with reference to the accompanying schematic and simplified drawings wherein
- Figure 1: is an oblique top view of a pass-through cabinet according to the invention, seen from its sample loading side,
- Figure 2: is a top view of the pass-through cabinet of Figure 1,
- Figure 3: is an oblique top view of a pass-through cabinet according to the invention, seen from its sample unloading side,
- Figure 4: is an oblique view of a pass-through cabinet according to the invention,
- Figure 5: is an oblique top view of a pass-through cabinet according to the invention, seen from the sample unloading side and with a wall of the equipment space of the cabinet removed,
- Figure 6: is a top and simplified view of the bottom of the equipment space of a pass-through cabinet according to the invention, and of its actuators, in a first position of the lock arrangement, as well as the doors of the pass-through cabinet without guides,
- Figure 7: is a top and simplified view of the bottom of the equipment space of a pass-through cabinet according to the invention, and of its actuators, in a second position of the lock arrangement, as well as the doors of the pass-through cabinet without guides, and

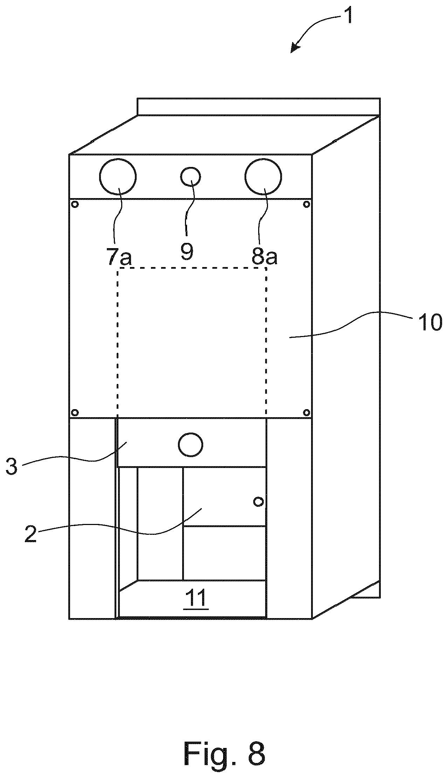

- Figure 8: is an oblique top view of a pass-through cabinet according to the invention, seen from the sample unloading side and in an abnormal situation, with both of its doors opened.

### DETAILED DESCRIPTION OF THE INVENTION

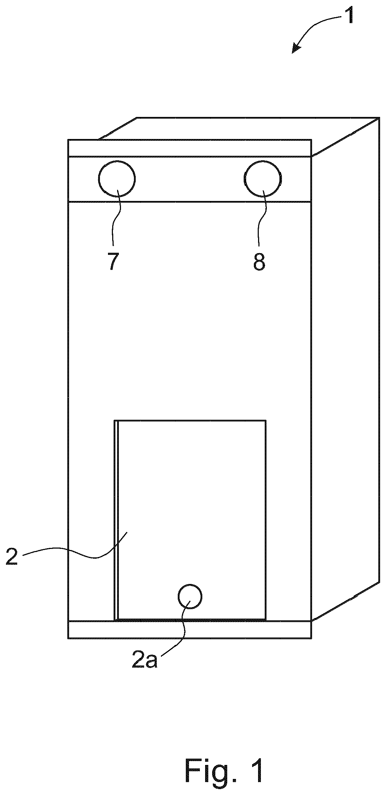

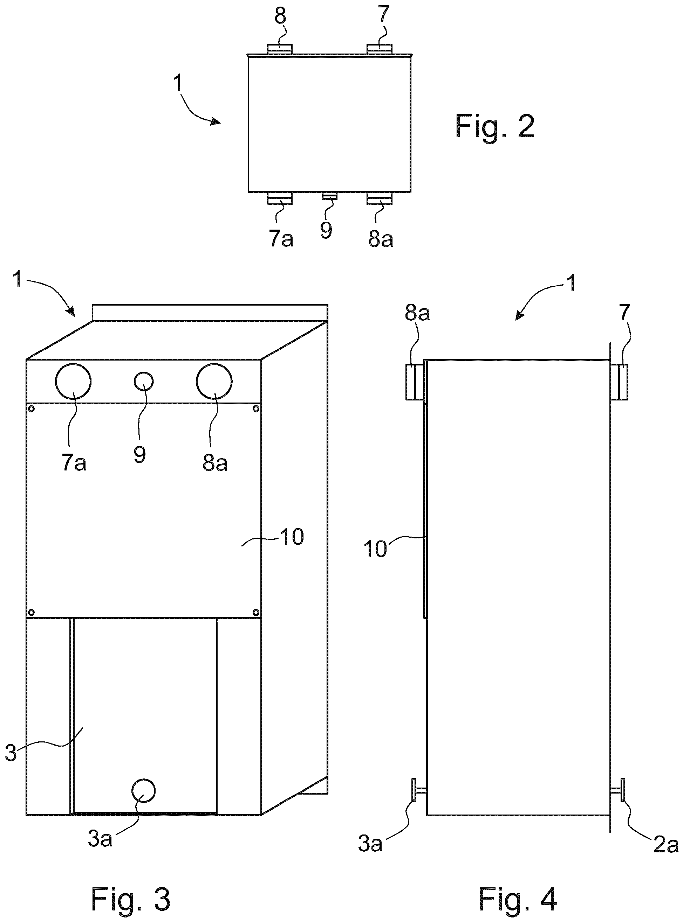

Figures 1 to 4 show a pass-through cabinet 1 according to invention and suitable for loading and unloading samples, seen in different directions from the outside of the cabinet. Preferably, the cabinet is a substantially rectangular cuboid in shape, for example, and comprises a cover, bottom, lateral walls as well as a front wall and a back wall, both of these having a substantially vertically movable sliding door. The pass-through cabinet 1 can stand alone at its location of use, or there can be more than one pass-through cabinets 1 in the same space. Preferably, the pass-through cabinet 1 is placed between two spaces, there being no other connection between these spaces but the connection provided by the pass-through cabinet 1.

Seen from the outside, the pass-through cabinet 1 shown in figures 1 to 4 has, on the front side of the cabinet, i.e. on the sample loading side, in the lower portion of the cabinet, a loading side door 2 openable by sliding the door 2 vertically upwards along guides. The door 2 is moved by lifting the door by a handle 2a along the sliding guides until the door is open up to a sufficient height. The door is maximally open when the handle 2a hits the upper edge of the door opening.

In addition, two indicator lights forming part of the indicator system of the cabinet are provided on the front side of the cabinet: a first indicator light 7 emitting a first color, preferably green, for example, and a second indicator light 8, emitting a second color, preferably red, for example. Both indicator lights 7 and 8 are connected to the control system of the cabinet and indicate the operating states of the pass-through cabinet 1.

On the back side, i.e. the sample unloading side of the pass-through cabinet 1, in the lower portion of the cabinet, an unloading side door 3, also openable by sliding the door 3 vertically upwards along guides, is provided. The door 3 is moved by lifting the door by a handle 3a along the sliding guides until the door is open up to a sufficient height. The door is maximally open when the handle 3a hits the upper edge of the door opening. The doors 2 and 3 can be substantially similar and movable in a substantially similar manner.

In addition, two indicator lights forming part of the indicator system of the cabinet are provided on the back side of the cabinet: a first indicator light 7a emitting a first color, preferably green, for example, and a second indicator light 8a, emitting a second color, preferably red, for example. Both indicator lights 7a and 8a are connected to the control system of the cabinet and indicate the operating states of the pass-through cabinet 1.

The back side of the cabinet is also equipped with a switching means 9, such as a press-button or a similar switching structure, forming part of the indicator system of the cabinet and also connected to the control system of the cabinet.

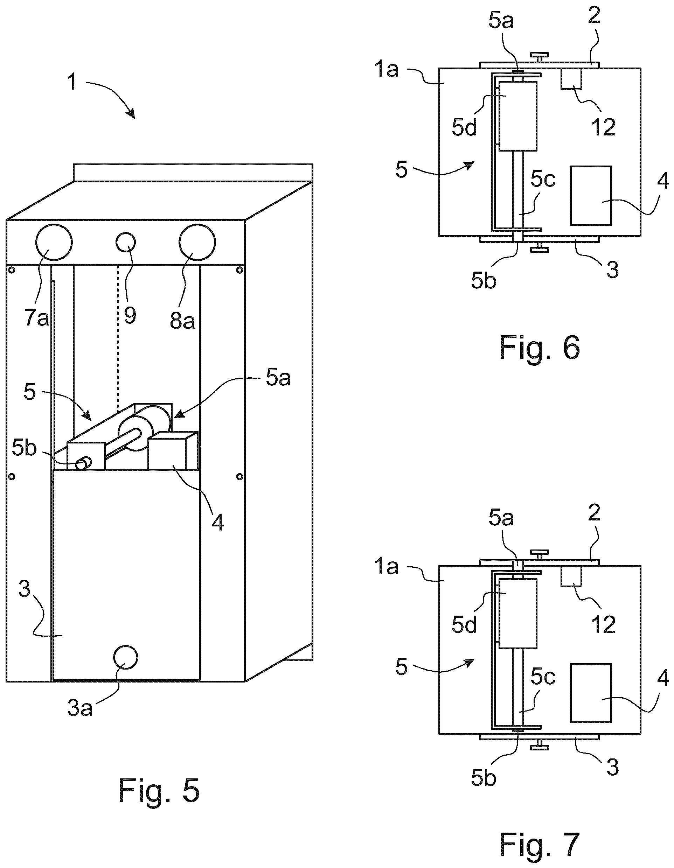

Figure 5 is an oblique front view of the pass-through cabinet 1 of figure 1, seen from the unloading side and without a front panel 10 in the upper portion of the cabinet. This makes it possible to see the equipment space, and its actuators, located in the upper portion of the cabinet and having a bottom 1a. The bottom 1a separates the sample space located in the lower portion of the cabinet 1 and the equipment space located in the upper portion of the cabinet from each other.

The pass-through cabinet 1 is has a control means 4 comprising a control system and coupled to co-operate with the loading side door 2, the lock arrangement 5, the indicator lights 7, 8, 7a and 8s as well as the switching means 9 of the cabinet 1. The pass-through cabinet can be either battery or mains powered. For clarity, the power switch or batteries are not shown in the figures. The control means 4 is adapted to control the operation of the lock arrangement 5 and indicator lights 7, 8, 7a and 8a of the cabinet by locking the loading side door 2 and switching on the red indicator lights 8 and 8a indicating the presence of a sample, once the loading side door 2 is closed, as well as by unlocking the loading side door 2 and switching on the green indicator lights 7 and 7a indicating that the cabinet 1 is vacant, as the switching means 9 is pushed down after taking out the sample and closing the unloading side door 3.

Figures 6 and 7 are simplified top views of the bottom 1a of the equipment space of the pass-through cabinet 1 according to the invention, and of its actuators. Figures 6 and 7 also show the doors 2 and 3 seen from above, but, for clarity, without the door guides and the wall structures of the cabinet.

In the situation shown in Figure 6, the unloading side door 3 is locked while the loading side door 2 is unlockable. Correspondingly, in the situation shown in Figure 7, the loading side door 2 is locked while the unloading side door 3 is unlockable.

The actuators of the equipment space include the control means 4, the lock arrangement 5 coupled thereto and a sensing means 12 monitoring the position of the loading side door 2 and also coupled to the control means 4. The sensing means 12 allows the loading side door 2 to be locked automatically and the unloading side door 3 to be unlocked at the same time as well as the operating state of the pass-through cabinet 1 to be indicated by means of the indicator lights 7, 7a, 8, 8a of the indicator system.

The control means 4 comprises the control system of the pass-through cabinet 1 and, if necessary, other actuating members monitoring and controlling the operations of the pass-through cabinet 1. The lock arrangement 5 comprises a body, an electric actuator 5d, preferably a solenoid and a rod-like locking member 5c, briefly a lockrod 5c whose first end 5a is adapted to lock, in a first position of the lock arrangement, the movement of the loading side door 2, as the first end 5a is moved to a position above the upper edge of the door 2, and whose second end 5b is adapted to lock, in a second position of the lock arrangement, as the second end 5b is moved to a position above the upper edge of the door 3. The sensing means 12, preferably a microswitch provided in connection with the loading side door 2, for example, is connected to the control system of the cabinet 1, preferably through the control means 4, as stated above.

Preferably, the actuators of the equipment space of the pass-through cabinet 1, or most of them, are adapted to form a single modular unit which, when doing updates, or, suffering a malfunction, is quick and easy to replace, for example, by disconnecting the module currently in use from the connector provided at the bottom 1a of the equipment space and by connecting a new module to it, just by pushing the module into place. The connector being already connected to the actuators of the cabinet 1, the cabinet is ready to operate immediately after the new module has been connected.

The doors 2 and 3 of the pass-through cabinet 1 are adapted to be manually movable in the vertical direction, for example, along groove-like guides provided on the inner surface of the front and back walls of the cabinet. The lock arrangement is double-acting in that the lockrod 5c only has two positions. In the first position, the second end 5b of the lockrod 5c is moved to a position above the upper edge of the unloading side door 3, leaving the unloading side 3 door locked and unmovable, while the loading side door 2 is open and freely movable upwards. In the second position, the first end 5a of the lockrod 5c is moved to a position above the upper edge of the loading side door 2, leaving the loading side 2 door locked and unmovable, while the unloading side door 3 is open and freely movable upwards.

The pass-through cabinet 1 according to the invention has an innovative indicator system allowing the cabinet to be used smoothly. As an example, indicator lights or other signals can be used for indicating which pass-through cabinet 1 is currently vacant for receiving a sample and which pass-through cabinet 1 contains a sample to be taken out.

As a person enters the sample collection facility to give a sample, the green indicators lights 7 and 7b are switched on on both sides of the cabinet 1 to indicate that the customer side, i.e. loading side 2, is not locked and that the sample space 11 is empty and can be used. The lockrod 5c has been moved to a position above the upper edge of the unloading side door 3, locking the unloading side door 3. This makes it impossible for the person giving the sample, or anyone else, to take anything away from the unloading side or to transfer anything to the unloading side, therethrough.

As the person giving the sample opens the loading side door 2 of the cabinet 1, puts the sample into the sample space 11 inside the cabinet and closes the loading side door 2 of the cabinet, the sensing means 12 reacts to the closing of the door 2 and the control means 4 activates the solenoid 5d to move the first end 5a of the lockrod 5c to a position above the upper edge of the loading side door 2, locking the loading side door 2 into its lower position. Now, the second end 5b of the lockrod 5c moves away from the position above the upper edge of the unloading side door 3 and the door 3 is unlocked, the green indicator lights 7 and 7a are switched off while the red indicator lights 8 and 8a are switched on to indicate that there is a sample in the sample space 11.

As the staff take out the sample on the unloading side and push the switching means 9, the control means 4 activates the solenoid 5d to move the second end 5b of the lockrod 5c to a position above the upper edge of the unloading side door 3, locking the unloading side door 3 into its lower position. Now, the first end 5a of the lockrod 5c moves away from the position above the upper edge of the loading side door 2 and the door 2 is unlocked, the red lights 8 and 8a are switched off while the green lights 7 and 7a are switched on to indicate that the sample space 11 is vacant and that door 2 can be opened again for inserting a new sample.

It will be appreciated by a person skilled in the art that the different embodiments of the invention are not solely restricted to the examples given above but may vary within the scope of the accompanying claims. Hence, the control means of the pass-through cabinet 1 according to the invention also may comprise some other type of switch than a microswitch. The control means may comprise a motion detector or a limit switch.

It will also be appreciated by a person skilled in the art that the pass-through cabinet 1 according to the invention can be mounted to stand freely on a table or platform or it can be installed in a wall between two rooms. There can be more than the pass-through cabinet in the sample collection facility.

It will also be appreciated by a person skilled in the art that the pass-through cabinet 1 according to the invention may comprise a sensing element provided in the sample space to sense the presence of a sample in the sample space, whereafter the loading side door is locked, while the unloading side door is opened, and the indicator lights indicating the current oper-ating state are switched on or some other signal indicating the current operating state is given.

It will also be appreciated by a person skilled in the art that the indicator lights of the indicator system may differ from the above. Thus, the lights of different colors indicating the respective operating state of the pass-through cabinet can be provided in the same lamp. It is just the color of the light that changes according to the operating state.

## Claims

1. A pass-through cabinet (1) comprising at least a loading side door (2), an unloading side door (3) and a lock arrangement (5) to lock and unlock the doors (2,3), **characterized in that** the pass-through cabinet (1) is equipped with an indicator system adapted to indicate the operating state of the pass-through cabinet (1).

2. A pass-through cabinet (1) as defined in claim 1, **characterized in that** the indicator system is adapted to indicate whether the pass-through cabinet (1) is vacant to receive a sample and an operating state where there is a sample in the sample space of the pass-through cabinet (1).

3. A pass-through cabinet (1) as defined in claim 1 or 2, **characterized in that** the indicator system comprises one or more first indicator lights (7, 7a) adapted to indicate that the pass-through cabinet (1) is vacant to receive a sample, and that the loading side door (2) of the pass-through cabinet (1) is unlocked.

4. A pass-through cabinet (1) as defined in claim 1, 2 or 3, **characterized in that** the indicator system comprises one or more second indicator lights (8, 8a) adapted to indicate that there is a sample in the sample space of the pass-through cabinet (1), and that the loading side door (2) of the pass-through cabinet (1) is locked and the unloading side door (3) is unlocked.

5. A pass-through cabinet (1) as defined in any of the receding claims, **characterized in that** the indicator system comprises a switching means (9) to lock the unloading side door (3), to unlock the loading side door (2) and to switch on the first indicator lights (7, 7a) indicating that the pass-through cabinet (1) is vacant as well as to switch off the second indicator lights (8, 8a).

6. A pass-through cabinet (1) as defined in any of the receding claims, **characterized in that** the pass-through cabinet (1) comprises a sensing means (12) to monitor the position of the loading side door (2).

7. A pass-through cabinet (1) as defined in any of the receding claims, **characterized in that** the pass-through cabinet (1) comprises a control system, the indicator system, the lock arrangement (5), the switching means (9) and the sensing means (12) being operatively coupled thereto.

8. A pass-through cabinet (1) as defined in claim 6 or 7, **characterized in that** the sensing means (12) is adapted to transmit a signal of closing the loading side door (2), to the control system, the control system being adapted to lock the loading side (2), to unlock the unloading side door (3), to switch off the indicator light (7, 7a) indicating that the pass-through cabinet (1) is vacant, on both the loading side and the unloading side of the cabinet, and to switch on the indicator light (8, 8a) indicating the presence of a sample, on both the loading side and the unloading side of the cabinet.

9. A pass-through cabinet (1) as defined in any of the receding claims, **characterized in that** the lock arrangement (5) comprises a body, an electric actuator (5d) attached to the body and a rod-like locking member (5c) moved by the actuator (5d), whose first end (5a) is adapted to lock, in a first position of the lock arrangement, the movement of the loading side door (2), and whose second end (5b) is adapted to lock, in a second position of the lock arrangement, the movement of the unloading side door (3).

10. A pass-through cabinet (1) as defined in claim 9, **characterized in that** the electric actuator (5d) of the lock arrangement (5) is a solenoid.
